Europäisches Patentamt

European Patent Office

Office européen des brevets

(19)

(11) Publication number: **0 329 306**

**A1**

(12) **EUROPEAN PATENT APPLICATION**

(21) Application number: **89301029.8**

(51) Int. Cl.4: **A61B 5/02**

(22) Date of filing: **02.02.89**

(30) Priority: **03.02.88 JP 21870/88**
**03.03.88 JP 48712/88**
**11.04.88 JP 47804/88**

(43) Date of publication of application:
**23.08.89 Bulletin 89/34**

(84) Designated Contracting States:
**CH DE FR GB LI SE**

(71) Applicant: **SECOM CO. LTD.**
**Shinjuku Nomura Bldg. 26-2, Nishi-Shinjuku**
**1-chome Shinjuku-ku**
**Tokyo(JP)**

(72) Inventor: **Takahashi, Hajume**
**c/o Secom Co. Ltd. 11-23 Shimorenjaku**
**6-chome**
**Mitaka-shi Tokyo(JP)**
Inventor: **Nakamura, Chikashi**
**c/o Secom Co. Ltd. 11-23 Shimorenjaku**
**6-chome**
**Mitaka-shi Tokyo(JP)**
Inventor: **Furukawa, Kenichi**
**c/o Secom Co. Ltd. 11-23 Shimorenjaku**
**6-chome**
**Mitaka-shi Tokyo(JP)**
Inventor: **Sato, Kenichi**
**c/o Secom Co. Ltd. 11-23 Shimorenjaku**
**6-chome**
**Mitaka-shi Tokyo(JP)**

(74) Representative: **Fisher, Bernard et al**
**Raworth, Moss & Cook 36 Sydenham Road**
**Croydon Surrey CR0 2EF(GB)**

(54) **Health care administration and measuring apparatus.**

(57) A health care administration and measuring apparatus having an armchair-like framework construction with a pair of arm sections, a seat, a back rest, and a base plate, and provided with a plurality of functional units including an operating unit accommodated in the back rest, and enabling a person to be examined to select and set measuring items necessary for measuring and examining his or her health, a measuring unit having a plurality of measuring ends accommodated in the arms and the base plate to measure the health care data, a control unit accommodated in the back rest to calculate the measured values of the health care data, and a display unit mounted on the uppermost face of the back rest to give visual and acoustic displays of the measured and calculated data of the health care administration as well as recording print-outs. The apparatus is also able to examine and determine a current physical condition and health on the basis of a comparison of the latest measured data with a specified standard range of the health care values of each measuring item.

EP 0 329 306 A1

# Fig. 1

# HEALTH CARE ADMINISTRATION AND MEASURING APPARATUS

## BACKGROUND OF THE INVENTION

### 1. Field of The Invention

The present invention relates to an apparatus for measuring an extend or adequacy of human physical conditions and health, mainly persons are presently considered to be in a normal physical condition, or healthy to enable a self administration of health care. More particularly, the present invention relates to a health care administration and measuring apparatus permitting a normally healthy person to easily and immediately measure his or her datum or data ( hereinafter referred to as health care data ), such as blood pressure, pulse, electrocardiogram, weight, temperature, and urinalysis, which can be used as parameters of the health of that person, by studying measurements obtained by an apparatus formed as a single unit. This apparatus is also able to display the measured health care data so enable the person to be acquainted with his or her actual physical condition at that time, and to conduct a statistical administration of health care by accumulating the health care data.

### 2. Description of the Related Art

A number of conventional medical care machines for determining a human physical condition with respect to individual health care datum, such as blood pressure, an electrocardiogram, pulse or weight, are used at hospitals, homes, and gymnastic or sport facilities.

With the conventional medical care machines, however, it is almost impossible to carry out measurements of health care data of a person at a constant measuring posture. Moreover, when the measurement is conducted in the presence of a doctor, it is difficult to obtain the measured data under a relaxed and normal psychlogical condition, due to an excessive tension of the measured person. Particularly, with respect to bleed pressure, if the measurement is not conducted under a related and normal psychlogical condition, it is almost impossible to acquire accurate measured data. Therefore, there is a strong demand for an apparatus capable of always measuring parameters for health car administration under a constant measuring condition.

In hospitals and clinics, a patient requiring a measurement of health care data usually must visit separate rooms specified for separate measuring items, and therefore, it takes a long time for the patient to finish the entire course of measurements, including waiting time. Consequently, the measurement of the health care data is very cumbersome, and accordingly, an apparatus for measuring health care data and capable of conducting the measurement of all measuring items at the same place and preferably at once is greatly desired.

In the conventional medical care machine, the normal values of data described in the instructions for the machine for a certain measuring item are used as indications of the adequacy of the patient's physical condition. Nevertheless, the normal values described in the instructions are always statistical averaged values, taking into account age and distinctions between males and females, and therefore, the normal values must be considered as defining a wide range of values. Further, when the medical care machine is used successively, the normal values in the instructions of the machine must be continuously used as criteria for the examination without taking into account changes in the operational reliability of the machine after a long usage thereof.

Practically, the normal values of human physical conditions vary from person to person, and thus the direct use of the above-mentioned statistical averaged values in the instructions of the conventional medical care machines is not accurate when examining the physical condition and health of each person. This is because the examination of the physical condition of each person and the administration of the health care of each person must be conducted by comparing the latest measured data with personal normal values or with the normal values in the instructions of the medical care machine, after updating same.

## SUMMARY OF THE INVENTION

Therefore, an object of the present invention is to provide a health care administration and measuring apparatus capable of obviating the above-mentioned defects of the conventional medical care machines and conducting the administration of personal health care individually for each person.

Another object of the present invention is to provide a health care administration and measuring apparatus formed as a single lightweight electronic unit able to measure health care data for a plurality of health care measuring items at the same time and at a constant measuring posture, under a normal psychlogical condition of the person measured by the unit.

A further object of the present invention is to provide a health care administration and measuring apparatus having an automatic compensating means periodically compensating for a zero-point deviation of a load cell component incorporated therein as a scale to measure the weight of a person, when the zero point value of the load cell component under no load gradually deviates from an initial value due to ageing of the load cell and due to a cyclic changes in an environmental temperature throughout the year.

A still further object of the present invention is to provide a health care administration and measuring network system in which one or more health care administration and measuring terminal units are interconnected by general control units, by utilizing a conventional telecommunication system or telephone lines, whereby a statistical determination of the adequacy of the physical or health conditions of a plurality of person can be carried out.

Yet another object of the present invention is to provide a health care administration and measuring apparatus capable of being intercommunicated with a personal computer owned by a medical doctor, to supply the doctor with health care data to be used for a medical examination of patients.

In accordance with one aspect of the present invention, there is provided a health care administration and measuring apparatus capable of measuring at least one health care data used as a parameter when examining the physical condition and health of a person under examination, the apparatus being characterized by comprising : an operating unit for selecting measuring items to be employed as said health care data for the person and setting same into the apparatus; a measuring unit interconnected with the operating unit and including a measuring end for measuring the health care data and generating output signals of measured data in response to a setting signal sent from the operating unit; a control unit for calculating measured values with respect to the health care data, on the basis of the output signals from the measuring unit; a data display unit for displaying the measured data with respect to the health care data to at least the person under examination; and a support unit for supporting the person under examination in a posture appropriate for the measureing, the supporting unit including a constructional framework member for accommodating and incorporating therein the above respective units.

In accordance with another aspect of the present invention, there is provided a health care administration system for the health care of a person by measuring more than one health care datum used as parameters of the physical condition and health of the person. The system comprises :
at least one terminal health caring and measuring unit which comprises an operating unit for selecting measuring items to be employed as the health care data for the person and setting same into the terminal health caring and measuring unit, a measuring unit interconnected with the operating unit, and including a measuring end to measure the health care data and generate ouput signals of measured data in responds to a setting signal issued from the operating unit, a control unit for calculating measured values with respect to the health care data on the basis of the output signals from the measuring unit, and a data display unit for displaying the measured data with respect to the health care data to at least the person under examination;
a determining unit for determining the adequacy of the physical condition of the person under examination on the basis of the measured values with respect to the health care data calculated by the control unit of the terminal health caring and measuring unit, the determining unit comprising: a data file interconnected with the terminal health caring and measuring unit and storing the measured values with respect to each of the selected items; a normal standard range calculating unit for calculating and setting a range of normal standard values from a plurality of past measured values of the person under examination with respect to each of the selected measuring items; and a comparing and determining unit for comparing the latest of the measured values with the range of normal standard values calculated and set by the standard range calculating unit.

According to the above-mentioned health care administration and measuring apparatus of the present invention, a person to be subjected to health care administration is able to select at least one item to be measured and obtain the measured health care data while held in a constant measuring posture on the apparatus in a relaxed normal psychlogical condition. The measured data is immediately indicated on the display unit so that the person and/or a medical examiner are immediately acquainted with the physical condition and health of the person at that time. A collection and storage of the health care data for administration are also achieved.

BRIEF DESCRIPTION OF THE DRAWINGS

The above and other objects, features, and advantages of the present invention will become more apparent from the ensuing description of the preferred embodiments with reference made to the accompanying drawings wherein:

Figure 1 is a perspective view of a health care administration and measuring apparatus according to an embodiment of the present invention;

Fig. 2 is a functional block diagram of the health care administering and measuring apparatus according to the present invention;

Fig. 3 is a block diagram of the apparatus of Fig. 1, illustrating the function thereof;

Figs. 4(a) through 4(d) are schematic and diagrammatic views illustrating different usage states of the apparatus of Fig. 1;

Fig. 5 is a block diagram illustrating an automatic compensating means for periodically compensating for a zero-point deviation of a load cell component used in the apparatus for measuring weight;

Fig. 6 is a schematic block diagram of a health care administration and measuring apparatus with an automatic zero-point compensating means according to an embodiment of the present invention;

Fig. 7 is a flow chart illustrating the operation of the automatic zero-point compensating means of the health care administration and measuring apparatus ;

Fig. 8 is a flow chart illustrating an interrupt routine of the automatic zero-point compensating operation;

Fig. 9 is a block diagram of a health care administration system having a plurality of terminal health care administering and measuring units accommodating therein according to the present invention;

Fig. 10 is a block diagram illustrating a determining unit of the system of Fig. 9;

Fig. 11 is a block diagram of a group standard range calculating means of the determining unit, illustrating the function thereof;

Fig. 12 is a diagrammatic view illustrating a standard value range with an average value $\mu \phi$ as the center thereof;

Fig. 13 is a flow chart illustrating the process of operation of a comparing and determining means of the health care administration system according to the present invention;

Fig. 14 is a block diagram of a determining unit of the health care administration system, according to another embodiment of the present invention;

Fig. 15 is a block diagram of a determining unit of the health care administration system, according to a furhter embodiment of the present invention; and

Figs. 16A and 16B are diagrammatic views, with the abscissa showing time, schematically illustrating the relationship between a group standard value range and a person standard value range.

DESCRIPTION OF THE PREFERRED EMBODIMENTS

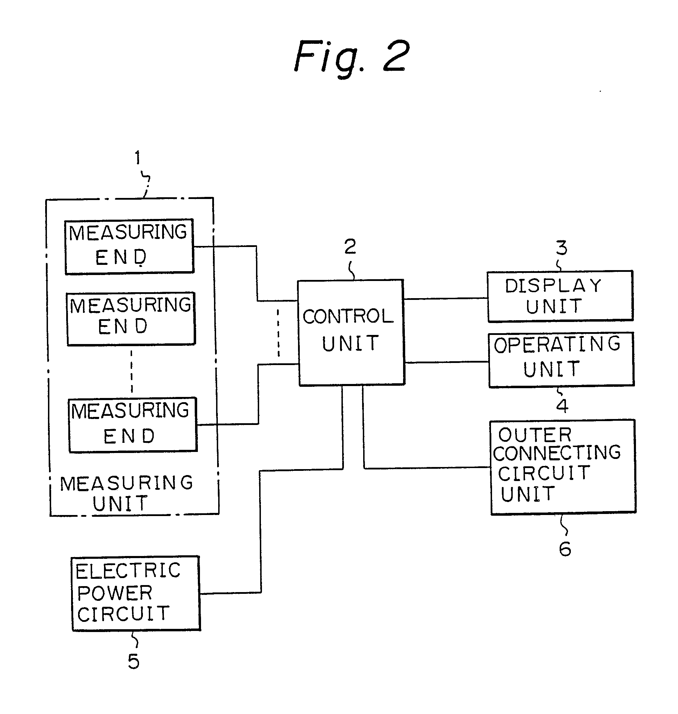

Referring first to Fig. 2, the health care administration and measuring apparatus according to an embodiment of the present invention includes a plurality of functional units, namely, a measuring unit 1 to measure health care items, such as blood pressure, pulse, electocardiogram, and weight, a control unit 2, a display unit 3, an operating unit 4, and an electric power circuit unit 5. In addition, the apparatus preferably includes an outer connecting circuit unit 6.

The measuring unit 1 has a plurality of measuring ends, mounted in or accommodated in sections of an armchair-like body of the apparatus, which are suitable for conducting measurements of the health care items. The armchair-like body of the apparatus is formed as a later-described constructional framework on which a person to be subjected to health care administration is able to be seated for the purpose of measuring health care data of the health care items.

The control unit 2 is assembled in an appropriate part of the body of the apparatus, such as inside a backrest of the armchair-like body, and operates to collect health care data of each normally healthy person under examination, which data is measured by the above-mentioned measuring unit 1 with respect to health care items, to be used as parameters indicating an adequacy of the physical condition and health of the person. The control unit 2 also calculates the measured and collected data to determine whether or not the physical condition and health of the person is adequate, and outputs the calculated data to the later-described display unit 3.

The display unit 3 comprises a sound synthesizing circuit, a liquid crystal display, and a printer, and

4

indicates the result of the health condition determined by the control unit 2, as visual images, voice outputs, and print-outs.

The operating unit 4 primarily comprises panel switches ( Note, other type of switches may be used. ), and is used for changing an operation mode of the health care administration and measuring apparatus as required, selecting measuring items for health care, selecting and registering an identification ( ID ) of each person to be examined. Selecting the type of display to be made on the display unit 3, calling out the data of measurement at the previuos time, starting and stopping the operation of the apparatus, and turning ON and OFF the electric power to the apparatus.

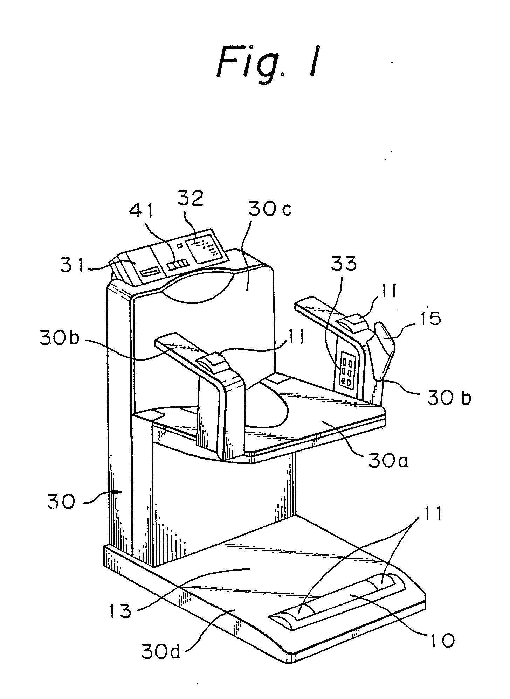

A description of the health care administration and measuring apparatus according to an embodiment of the present invention will be hereinafter provided with reference to Figs. 1 through 4.

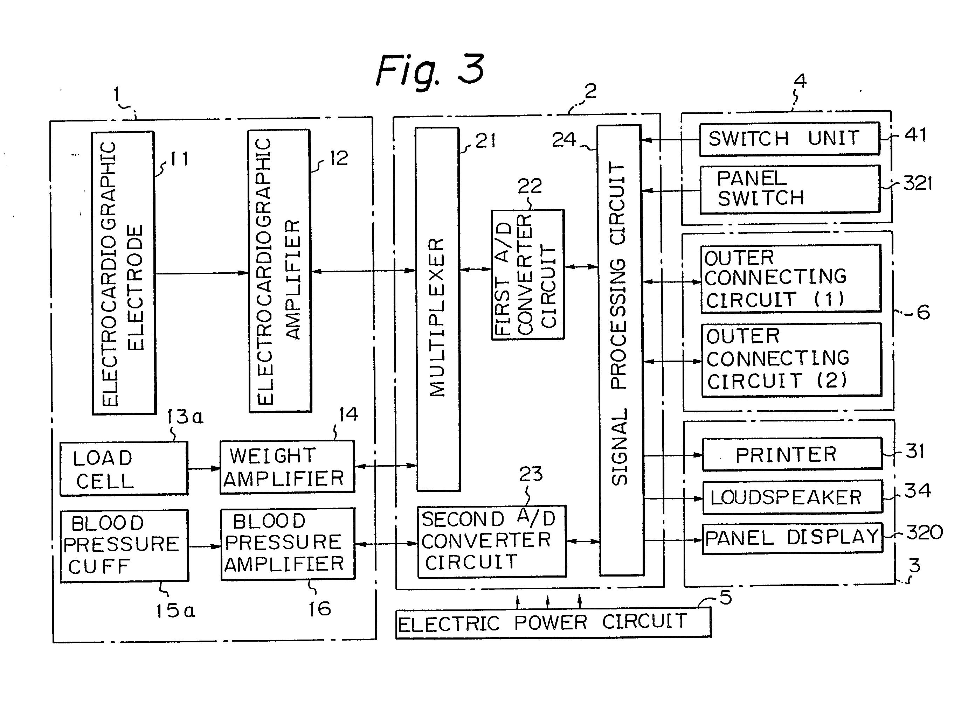

Figure 1 illustrates an embodiment of the health care administration and measuring apparatus, which is constructed in the shape of an armchair. The apparatus is provided with a printer 31 of the display unit 3, for printing out results from the later-described control unit 2, automatically or as instructed. The apparatus also has a touch-switch panel 32, which includes an LCD ( liquid crystal diode ) panel display 320 shown in Fig. 3 and panel switches 321 to change and set the operation mode of the health care administration and measuring apparatus.

The apparatus is also provided with indicating lamps 33 which are lit to ensure that the respective physical measured parts of a person to be examined are placed exactlt at the positions of the measuring unit 1 suitable for such measurements, electrocardiographic electrodes 11 to be attached to the physical parts of the person for an electrocardiogram, a scale 13, and a finger type hemodynamometer, located in the armchair-lie body and covered with a covering 15. These electrodes 11, scale 13, and hemodynamometer covered by the covering 15 are all measuring ends of the measuring unit 1. A section of the apparatus designated at 41 is an assembly of switches of the operating unit 4, and these switches are used for turning the electric power ON and OFF, starting and stopping the measuring operation of the apparatus, applying an input signal to feed a printing paper of the printer 31, and turning the voice output ON and OFF. The above-mentioned functional units are accommodated in a body 30 of the apparatus, which includes a seat 30a on which a person to be measured can be seated for measuring, a pair of arms 30b supporting the arms of the person, a backrest 30c, and a base plate 30d having a scale 13 therein.

Figure 3 illustrates the detailed functional arrangement of the embodiment of Fig. 1.

As illustrated in Fig. 3, the measuring unit 1 has an electrocardiographic amplifier 12 connected to the afore-mentioned electrocardiographic electrodes 11 to detect electrocardiographic signals measured by the electrodes 11 and to transmit the measures electrocardiographic signals to the next stage, a load cell 13a forming the afore-mentioned scale and outputting an electric signal indicating a measured weight, a weight amplifier 14 to receive the electric signals from the load cell 13a for generating a corresponding weight signal to be output, a blood pressure cuff 15a, which is an example of the afore-mentioned hemodynamometer, and a blood pressure amplifier 16 to amplify electric signals supplied from the blood pressure cuff 15a.

The control unit 2 has a multiplexer 21 for receiving the electrocardiographic signals supplied from the electrodes 11 via the electrocardiographic amplifier 12 and the electric weight signals supplied from the load cell 13a via the weight amplifier 14, and converting these signals to corresponding output signals, a first analogue to digital ( A/D )converter circuit 22 connected to the multiplexer 21, a second analogue to digital converter circuit 23 connected to the blood pressure amplifier 16 to carry out an A/D conversion of the measured electric blood pressure signals supplied from the blood pressure cuff 15a via the amplifier 16, a signal processing circuit 24 including therein a CPU, a ROM, and a RAM, and processing, calulating and controlling of all signals supplied therein.

The afore-mentioned display unit 3 has a printer 31, the afore-mentioned panel display 320, and a loudspeaker 34.

The operating unit 4 has a switch unit 41 and a panel switch 321, and is used for starting the measuring operation, setting one of the operation modes in the apparatus, and indicating the setting method and the setting results.

The electric power circuit 5 is connectable to a commercial power source, e.g., an AC power source at 100 volts, and 50 or 60 hertz, and is arranged such that electric power is supplied to all of the above-mentioned units. The circuit 5 is disposed at a suitable position on the bode 30 of the apparatus.

Preferably, an outer connecting circuit unit 6 having outer connecting circuits ( 1 ) and ( 2 ) is further accommodated in the body 30 of the apparatus for receiving input signals sent from outer measuring devices, such as a measuring device for urinalysis to be used privately, and an outer electric thermometer, and for transmitting the signals of the measuring result to, for example, a separate outer memory unit, to accumulate therein the measuring results as one of the past measured data.

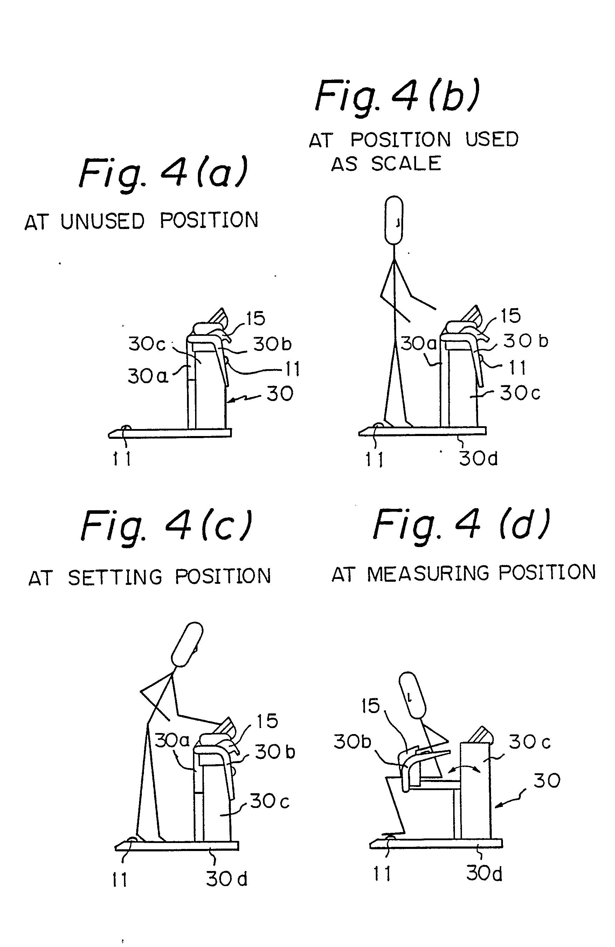

The operation of the health care administration and measuring apparatus according to the above-mentioned embodiment and the method of using the respective functional units will now be provided with reference to Figs. 4 a through 4d.

The armchair like body 30 of the apparatus can be collapsed into a compact state, as illustrated in Fig. 4a, when not in use, by turning the seat 30a and the pair of left and right arms 30b toward the backrest 30c. Namely, the seat 30a rests against the backrest 30c to thereby define an open space before the backrest 30c. Therefore, a person to be examined can stand on the base plate 30d having the scale 13 therein to conduct a measurement of weight, as illustrated in Fig. 4b. The weight measurement also may be carried out when seated, as illustrated in Fig. 4d by sitting on the seat 30c. When the apparatus of the present embodiment is energized by the afore-mentioned electric power circuit 5, and when a predetermined load is applied to the load cell ( the scale ) 13, the display unit 3 is also automatically energized to indicate the measured value on the display unit 3 as a voice output, visual image or print-out.

The method of measuring health care data will now be described.

Referring to Fig. 4c together with Figs. 1 and 3, the person to be examined stands on the base plate 30d of the bode 30 of the apparatus, collapsed as illustrated in Fig. 4c, to operate the switch unit 41 located on the upper margin of the backrest 30c. Namely, the person presses one of the starting and stopping switches of the switch unit 41 while standing erect. The starting signal is fed from the switch unit 41 to the signal processing circuit 24, which in turn commands a change of the operation mode of the apparatus from a stand-by mode to a measuring mode. Note : when only a turn-ON of the apparatus command is manually given from the switch unit 41 of the operating unit 4, the operation mode of the apparatus is maintained at the stand-by mode. The signal processing circuit 24 then supplies a signal, indicating that the measuring mode of the apparatus is started, to the display unit 3 to thereby provide the person to be examined with visual information for the start of the measuring mode on the panel display 320 and/or similar voice output information from the loudspeaker 34. Therefore, the person to be examined per se ( or an operater in some cases ) selects its own name from a plurality of registered names indicated on the panel display 320, by manually operating the operating unit 4 or by inputting a code identification number designating the person to be examined.

In the case of the present embodiment, the above-mentioned name selection operation is easily conducted by manually touching a desired one of the touch-panel switches 321, which are arranged in a part of the panel display 320 and capable of displaying the registered names. The selected name is transmitted from the operating unit 4 to the signal processing circuit 24, which in turn outputs a command signal to the display unit 3 to display the measuring items, with respect to the health care administration, on the panel display 320. Thus, a plurality of measuring items, such as blood pressure, cardiogram, pulse, weight, temperature, and urinalysis are displayed on the panel display 320 in response to the command signal from the signal processing circuit. The person to be examined then again operates the panel switches 321 of the operating unit 4 to select one or more measuring items from the displayed items. Note: the panel switches 321 include those for use in calling out the measured data of the previous time as required.

It is now assumed that the blood pressure, cardiogram and weight items are selected by the person to be examined. These measuring items selected by operating the panel swiches 321 are output to the signal processing circuit 24.

The person to be examined then sits on the seat 30a ( see Fig. 4d ) of the armchair-like body 30 to conduct the measurement of the blood pressure, cardiogram, and weight. At that time, the person places naked feed on the electrodes 11 on a foot rest 10 arranged in a front section of the base plate 30d, and inserts a forefinger of the left hand into the blood pressure cuff 15a ( Fig. 3 ) from an entrance of the covering 15 ( see Fig. 1 ). The person to be examined attaches pectoral electrodes ( not illustrated in Figures ) stored in the body 30 to the breast region as required, and further, locates its left and right wrists on the cardiographic electrodes 11 of the arms 30b of the bode 30. The pectoral electrodes can be easily attached to the breast region by the use of conventional sucker devices, and the measured signals of the pectoral electrodes are sent to the signal processing circuit 24 of the body 30 by inserting the electric terminals of the pectoral electrodes into connecters arranged on the left arm 30b. The location of the feet and wrists of the measured person on the exact positions and in contact with the electrodes 11 can be confirmed by the lighting of the indicating lamps 33 comprised of LEDs. When the LEDs are green, it is confirmed that the feet and wrists are correctly located on the electrodes 11. Conversely, when the feet and/or wrists are not correctly located on the determined positions of the electrodes 11, the indicating lamps intermittently flash red, to promote the relocation of the feet and/or wrist on the exact positions of the electrodes 11. Thus, the measured person can easily determine whether or not the feet and the wrists are at the exact positions of the cardiogrphic electrodes 11.

6

When the entire preparation for measurement of the person is completed, the signal processing circuit 24 sends a measurement start signal to each of the measuring ends of the measuring unit 1, which are used for measuring the above-mentioned selected measuring items.

In the present embodiment, the apparatus has two signal transmitting lines illustrated in Fig. 3, i.e., one for an elecrocardiogram and weight, and the other for blood pressure. The measurements of the elecrocardiogram, weight, and blood pressure are simultaneously conducted.

The measuring principle of the blood pressure employed in the present embodiment will be briefly explained herein below.

The measuring principle is based on a well known photoelectric plethysmographic method measuring volume pulse wave by applying a pressure supplied from a pressurizing pump to a finger of the person under examination through a pressing blood pressure cuff. When the pressure is applied, the inner volumes of the blood vessels of the finger change due to a difference between the applied pressure and the inner blood pressure, and therefore, the volume changing component corresponding to a pulse wave component of the blood pressure also simultaneously changes, depending on the difference between the applied pressure and the inner blood pressure, due to a strong non- linearity of the photoelectric to volume characteristics of the blood vessels. At this stage, when the finger wrapped by a blood pressure cuff 15a is placed between a light emitting element ( e.g., a LED element ) and a light receiving element ( a photo-transister ), the applied pressure is initially increased to an extent such that the pulse wave becomes extinct, and subsequently, the applied pressure is gradually decreased. As a result, the light receiving element, i.e., the photo-transister, generates an output including an alternating current component at a given pressure point. Then, the width of the alternating current component gradually increases to the maximum value, and thereafter, decreases. Thus, during the above-mentioned process, a change in the height of the photoelectric pulse wave is processed by a central processing unit ( CPU ) of the signal processing circuit 24 to thereby calculate the value of the blood pressure of the measured person. Note : the above-mentioned measuring principle per se is well known in the field of the medical care.

The blood pressure signals measured by the blood pressure cuff 15a are supplied to the blood pressure amplifier 16 in which an average value of blood pressure and the maximum value of the blood pressure are detected. These two values are then sent to the second A/D converter circuit 23 as the blood pressure measuring signals, and converted into corresponding digital signals which become digital inputs to the signal processing circuit 24.

Measurement of the weight of the examined person is carried out by the load cell 13a accommodated in the base plate 30d of the armchair like body 30 of the health care administration and measuring apparatus. The weight of the person sitting on the armchair is measured by the load cell 13a as an additional weight to the basic weight of the body 30 of the apparatus per se. Thus, the measurement of the weight can be achieved simultaneously with measurements of the other measuring items. The load cell 13a generates an electric weight signal corresponding to the measured weight of the person, and sends the signal to the weight amplifier 14. The weight amplifier 14 in turn detects the measured weight data signal from the electric weight signal, and transmits it to the signal processing circuit 24 via the multiplexer 21 and the first A/D converter circuit 22. It should be appreciated that the multiplexer 21 is arranged for discriminating the difference between the weight data signal and the elecrocardiogram data signal. The first A/D converter circuit 22 is arranged for converting the signals from the two amplifiers 12 and 14 into corresponding digital signals able to be processed in the signal processing circuit 24.

The electrocardiogram of the examined person is measured by the electrocardiographic electrodes 11 attached to the soles of the naked feet, the wrists and the breast region. These electrodes 11 detect a first through third and a breast-induced electrocardiographic complexes, which are in turn converted by the electrocardiographic amplifier 12 into an electric cardiographic signal. This signal is sent to the signal processing circuit 24 via the above-mentioned multiplexer 21 and the frst A/D converter circuit 22.

When the signal processing circuit 24 receives the above-mentioned blood pressure digital signal, the weight digital signal and the electric cardiographic digital signal sent from the measuring unit 1, the circuit 24 performs data processing of these digital signals with respect to each of the measuring items. When completed, the signal processing circuit 24 transmits a signal indicating the completion of the data processing to the display unit 3. Therefore, the display unit 3 informs the examined person of the completion of the measuring operation through the loudspeaker 34.

The signal processing circuit 24 then discriminates the measured results and outputs the results to the display unit 3, where the results are displayed on the panel display 320 with respect to each of the measuring items in such a manner that the result of each measuring item is continuously and automatically scanned. When the results should be printed by the printer 31, a print switch provided in the panel switches 321 is manually pressed so that an automatic printing of the results is carried out.

Although not selected by the measured person, when the temperature of the person is to be measured, a sublingual thermometer ( not shown )is taken out of a container formed in the apparatus body 30, and the terminal of the thermometer opposite to a measuring terminal is inserted into an electric connecter arranged in one of the arms 30b. The measured temperature signal is sent to the signal processing circuit 24 in a manner similar to the above-mentioned signals.

When the measured person visually or acoustically confirms the results of the measurements on the display unit 3, the start/stop switch of the operating unit 4 is pressed to stop the measuring mode of the apparatus. Thus, the operation of the apparatus is changed from the measuring mode to the stand-by mode. In the stand-by mode of the apparatus, it is possible to conduct a measuring of only the weight.

In the present embodiment, since the health care administering and measuring apparatus includes the outer connecting circuit unit 6 capable of receiving signals from the outside and outputting signals to the outside, the measuring data of, e.g., the urinalysis, conducted outside the apparatus, such as in a designated test area, can be processed by the signal processing circuits 24 of the apparatus when the data is input the apparatus through the outer connecting circuit unit 6.

The provision of the outer connecting circuit unit 6 also permits the afore-mentioned processed measured data together with an ID code of the person and a contract code to be sent via conventional communication lines to an outer elctronic processing unit or installation, to be further processed by the outer electronic processing unit. For example, when the outer electronic processing unit has a data file for effecting a statistical processing of the data by the use of filed standard values or standard ranges of respective health care measuring items, it is possible to compare the afore-mentioned data sent from the apparatus of the present embodiment with the filed standard values of ranges to thereby determine whether or not the physical condition and health of the examined person is adquate.

On the other hand, when either a personal standard range of each person to be measured with respect to each of the measuring items on the basis of the past measurements or a normal standard range of each of the measuring items depending on age and differences between male and female is preliminarily registered in a memory means of the circuit 24, it is possible to compare the latest measured data with the personal or normal standard range, and to display the results of the comparison on the display unit 3 together with a brief message such as " the latest data are within the personal standard range. ", " the data are within the normal standard range " or " please consult a medical docter ".

During the above-mentioned comparing operation carried out either outside or within the apparatus, the display unit 3 displays a message, for example, " the analysis of the latest measured data is now being processed. " The data sent to the outer processing unit may be stored therein as past health care data of that person.

When the data comparison is carried out by the outer installation, the result of the comparison can be sent back to the apparatus of the present embodiment so that the result may be indicated to the examined person.

When the outer unit or installation is a personal computer owned by a medical doctor, the measured data may be utilized by the doctor to conduct a medical examination and to make a clinical recording of that person.

In the present embodiment, the body 30 of the apparatus has the shape of an armchair, but may have the shape of a clinical bed.

Preferably, a plurality of siganal processing circuits 24 are arranged so that the individual circuit 24 can separately process each of the plurality of health care measuring items, to thereby achieve an immediate data processing. Then, the measuring and discrimination of respective measured data can be conducted at the same time.

From the foregoing description of the above-mentioned embodiment, it will be understood that the measurements of the parameters used as the health care data can be always carried out by the apparatus of the present invention under a relaxed psychlogical condition and a constant measuring posture of a person to be examined at a determined measuring position of the apparatus, and therefore, the measured data is actual and highly reliable for the administration of the health care of a person. Accordingly, the decision of the adequacy of the physical condition and health of the person can be very accurate and high quality.

When the physical condition and health is considered not adequate, and a disease for example, exists therein, since a message advising the person to consult a physician is displayed, it is possible to obtain an early detection of an illness and therefore, a self-care of personal health can be achieved.

Further, according to the health care administration and measuring apparatus of the present invention, since simultaneous and rapid measurements of a plurality of health care data can be carried out at a high efficiency, only a minimum waiting time is needed, and thus, no time is wasted.

Since the health care administration and measuring apparatus of the present invention is formed as an integral framework unit, it may be used at home and conduct the measuring of the health care data simply and easily. When conducting such measurements frequently, it is possible to present valuable data that can be used by a medical docter for examination of the health of a person, and thus, a high quality health care administration can be achieved.

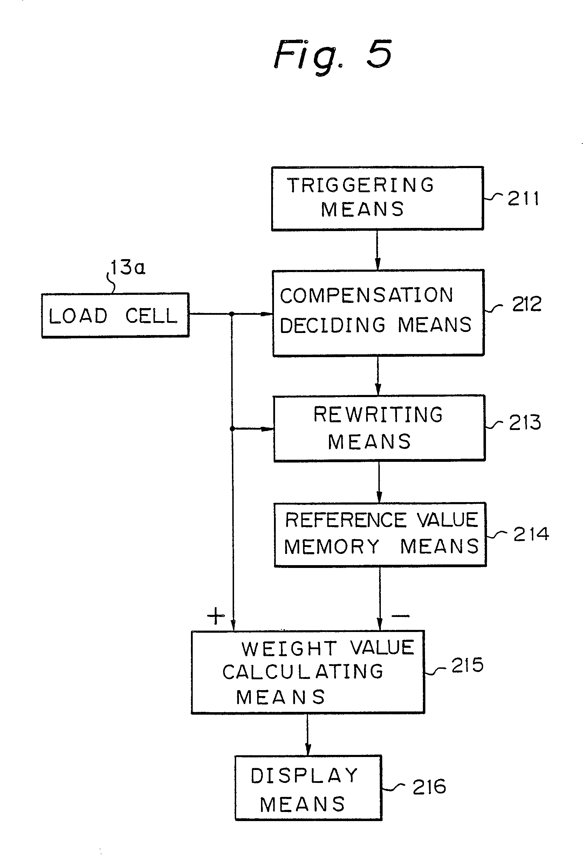

Figure 5 illustrates another functional means provided for a health care administration and measuring apparatus accoding to the present invention, i.e., an automatic zero point compensating means to compensate for a deviation of the no load reference value of the load cell 13a, which changes depending on age and a change in an environmental temperature during the year. Note: the no load reference value is an output of the load cell 13a when no load except for a load of a machine weight per se is added to the load cell 13a.

In Fig. 5, the automatic zero point compensating means to compensate for the no load reference value of the load cell 13a, used as a scale for measuring a weight of a person, includes a triggering means 211 for generating a compensating operation triggering signal at a predetermined time period, preferably a predetermined time of a calendar day, a compensation deciding means 212 arranged to receive the compensating operation triggering signal of the triggering means 211 for deciding whether or not a difference between an output of the load cell 13a at the present time and the no load reference value of the load cell 13a which is stored at the present time in a reference value memory means 214 is maintained within a predetermined range, a rewriting means 213 for rewriting the no load reference value stored in the reference value memory means 214 with the value of the output of the load cell 13a at the present time when the above-mentioned difference is decided to be within the predetermined range, a weight value calculating means 215 for calculating an actual weight of a given load, i.e., calculating a difference between an output weight signal of the load cell 13a issued when loaded by the given load and the no load reference value of the reference value memory means 214 where no compensation triggering signal is generated from the triggering means 211, and a display means 216 to display the calculated actual weight value of the given load applied to the load cell 13a.

The above-mentioned triggering means 211 may comprise a timer device set so that a compesation triggering signal is generated therefrom at a predetermined time, for example, at a predetermined time early in the morning of each calendar day. That is, the signal generating time is chosen to be a time when load cell 13a is not usually used for weighing. Alternatively, the triggering signal may be generated by an appropriate software.

When a load, such as the weight of a person to be measured, is not loaded on the load cell 13a, an actual electric signal currently output from the load cell 13a is received by the compensation deciding means 212, whereat the actual signal under no load is compared with a predetermined range of value for compensation. When the compensation deciding means 212 decides that the actual signal is deviated from the predetermined range of value for compensation due to, for example, age or an environmental temperature change, it generates a rewriting command signal to the rewriting means 213. As a result, the rewriting of an old no load reference value stored in the reference value memory means 214 as a fresh no load reference value corresponding to the value of the actual signal outputting from the load cell 13a, is carried out.

When a compensation triggering signal is not sent from the triggering means 211, the load cell 13a generates an electric signal corresponding to the sum of the weight value of a load applied to the load cell 13a, i.e., the weight value of a person, and the fresh reference value used for rewriting the reference value memory means 214, and inputs the electric signal into the weight value calculating means 215, whereat the fresh reference value supplied from the no load reference value memory means 214 is subtracted from the above-mentioned sum of the weight value of the load and the fresh reference value to calculate the actual weight of the load corresponding to an actual weight of the person. The actual weight of the person is then displayed on the display unit 3.

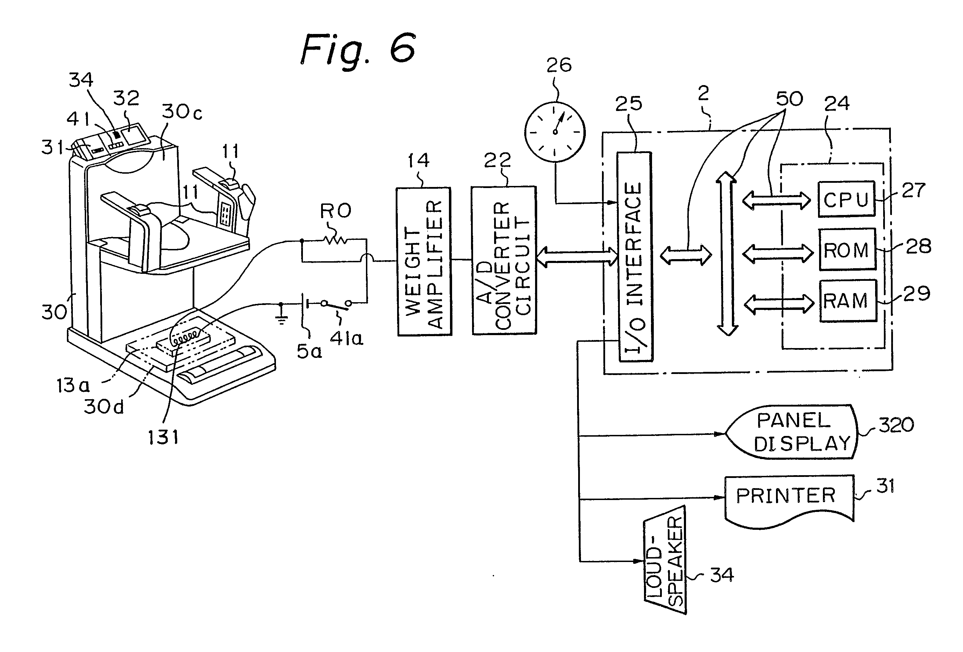

Figure 6 schematically illustrates a health care administration and measuring apparatus with the above-mentioned automatic zero point compensating means according to another embodiment of the present invention, wherein the same or like units or elements as in the afore-mentioned embodiment of Fig. 1 through 3 are designated by the same reference numerals.

Referring to Fig. 5 together with Fig. 6, a body 30 of the apparatus is in the form of an armchair having a base plate 30d in which a load cell 13a used as a scale is accommodated, and a backrest 30c in which a CPU of a control unit 2 and associated electric circuits are incorporated. On the uppermost face of the backrest 30c of the body 30 are mounted an assembly of touch-panel switches 32, a printer 31, an assembly of manually operated switches 41, and a loudspeaker 34 capable of providing sound information of a weight measured.

9

The load cell 13a of the base plate 30d of the armchair like body 30 includes a strain gauge 131 and a later-described electric reference resistor RO. The strain gauge 131 changes an electric resistance thereof depending on a load on the load cell 13a via the base plate 30d. When a predesigned electric power from an electric power source 5a is applied to the load cell 13a via a switch 41a of the assembly of manual switches 41 and the reference electric reference resistor RO, an electric voltage depending on the value of an electric resistance of the strain gauge 131 can be taken from the load cell 13a. The electric voltage is then amplified by a weight amplifier 14, and is supplied, via an A/D converter circuit 22, to an I/O interface 25 of the control unit 2 to which a separate timer 26 is also connected, to supply the control unit 2 with the afore-mentioned compensation operation triggering signal at a predetermined time period. Namely, the timer 26 is provided as the triggering means 211 ( Fig. 5 ).

The control unit 2 includes a signal processing circuit 24 provided with a CPU 27, a ROM 28, a RAM 29, and data buses 50, in addition to the above-mentioned I/O interface 25. The CPU 27, the ROM 28, the RAM 29, and the data buses 50 of the signal processing circuit 24 exhibit the functions of the above-mentioned compensation deciding means 212, the rewriting means 213, the reference value memory means 214, and the weight value calculating means 215. The CPU 27 also functions as a timer. Therefore, the CPU 27 instead of the above-mentioned separate timer 26, is able to generate the compensation operation triggering signal. The control unit 2 is able to conduct a later-described processing of the weight measuring data and to permit the panel display 320, the loudspeaker 34, and the printer 31 to indicate the weight measuring data for the measured person.

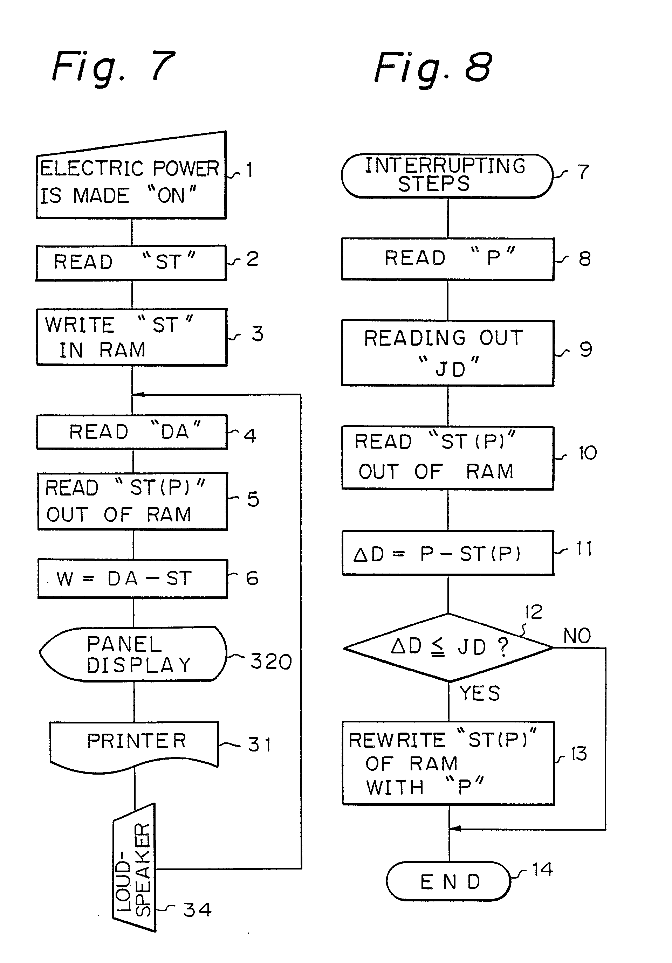

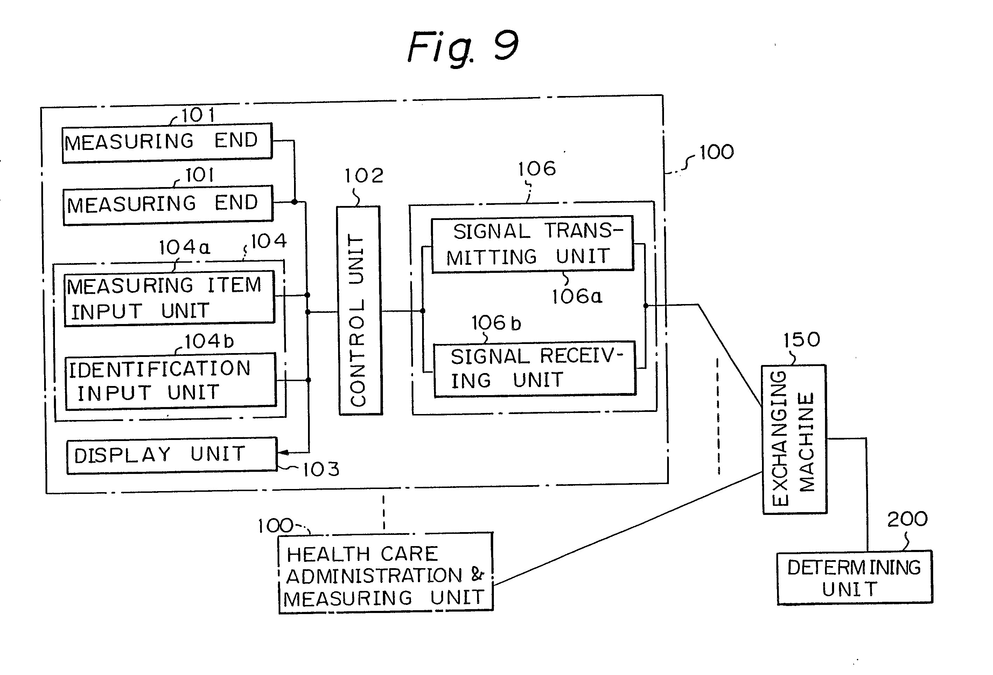

The description of the automatic zero point compensating operation for the load cell 13a carried out by the control unit 2 will be provided hereinbelow with reference to Figs. 7 and 8.

First, consideration will be given to the start of the operation of the load cell 13a. At step 1, the switch 41a is manually operated to turn ON the electric power supply from the electric power source 5a. The manual operation of the switch 41a also turns on the power line to the controlling unit 2. The strain gauge 131 of the load cell 13a is electrically energized.

At this stage, a predesigned condition exists such that, at the moment of operation of the manual switch 41a, no load should be loaded onto the base plate 30d. Therefore, an electric signal input to the I/O interface 25 of the control unit 2 from the load cell 13a via the weight amplifier 14 and the A/D converter circuit 22 indicates the no load reference value of the load cell 13a at the present time. The no load reference value gradually changes, due to age, from an initial preset reference value that has been set during manufacturing of the load cell 13a.

The no load reference value of the load cell 13a at the present time, i.e., at the moment of switching the switch 41a, is received by the I/O interface 25 of the control unit 2 as a value of an elecric voltage signal " ST " indicating an actual no load reference value of the load cell 13a at step 2.

The value " ST " indicating the actual no load reference value at the present time is subsequently written in the RAM 29 so as to be temporarily registered therein at step 3.

When a load of more than a predetermined value is loaded onto the base plate 30d, an output " DA " of the load cell 13a is sent, and read by the control unit 2, via the weight amplifier 14 and the A/D converter circuit 22 at step 4.

Subsequently, the above-mentioned value " ST " indicative of the actual no load reference value of the load cell 13a is read out of the RAM 29 at the step 5, and is sent to the CPU 27, where the calculation of $W = DA - ST$ is carried out at step 6. The value " W " indicates the value of the load given to the load cell at the present time. The value " W " is output to the panel display 320, the loudspeaker 34, and the printer 31 of the display, respectively, and the processing operation returns to step 4.

The above-mentioned starting operation of the load cell 13a controlled by the controlling unit 2 is insufficient for compensating a deviation of the no load reference value of the load cell 13a from the initial predesigned value due to age and a change in an environmental temperature during the year, and therefore, a series of interrupting steps shown in Fig. 8 is conducted by the control unit 2. Namely, at step 7, the interrupt routine is commenced upon receipt of the compensation operation triggering signal sent from, e.g., the timer 26 ( Fig. 6 ) at the predetermined time for example, at 3 o'clock in the morning on each calendar day. Note: the switch 41a is maintained at the " ON "position.

An output singal " P " sent from the load cell 13a via the weight amplifier 14 and the A/D converter circuit 22 is received and read by the control unit 2 at step 8, and then a comparing value " JD " preliminarily registered in the ROM 28 is read out thereof. The comparing value " JD " indicates a predetermined value on which a decision is made by the CPU 27 about whether the compensation of the no load reference value of the load cell 13a is necessary, and the value " JD " corresponds to, for example, 0.5 Kg.

At step 10, the afore-mentioned actual no load reference value " ST " is read out of the RAM 29, and a

calculation : $\Delta D = P - ST$ is carried out to obtain the differential $\Delta D$ at step 11.

Subsequently, a comparison of the differential $\Delta D$ with the comparing value " JD " is carried out to determine whether or not $\Delta D$ is less than the value " JD " . Note: $\Delta D$ may be a minus value. This comparison is made to determine whether or not a difference $\Delta D$ between the present output " P " of the load cell 13a and the registered no load reference value " ST ", which is the past no load output from the load cell 13a, is less than the comparing value " JD ". If the difference $\Delta D$ is less than the comparing value " JD ", i.e., less than 0.5 Kg, it is determined that an additional load, such as an article or a person, is not loaded onto the base plate 30d of the body 30 of the apparatus, and the no load reference value has drifted from the initial predesigned value due to age or a temperature change. Accordingly, the no load reference value " ST " registered in the RAM 29 at the step 3 of Fig. 7 is rewritten with the present actual output " P " from the load cell 13a at the step 13. When the rewriting of the no load reference value of the load cell 13a is completed, the interrupt routine is terminated at step 14.

On the other hand, when $\Delta S > JD$, i.e., when a difference between the present actual output " P " of the load cell 13a and the no load reference value " ST " corresponding to the past no load output of the load cell 13a is larger than 0.5 Kg, it is determined than an additional load is applied to the base plate 30d of the apparatus body 30, and the rewriting of the no load reference value of the RAM 29 is not carried out.

The above-mentioned interrupting steps 7 through 14 for compensating the no load reference value ( zero point ) of the load cell 13a of the health care administration and measuring apparatus of the present embodiment is periodically repeated every day in response to a compensation triggering signal outputting from the timer 26 at the predetermined time, i.e., at 3 o'clock on each calendar day. Therefore, when the value " ST " of the RAM 29 is rewritten with the value " P " on a given day, the value read out of the RAM 29 at step 10 corresponds to the value " P " on the day prior to that given day. This means that, on the given day, the value " P " on the prior day is replaced with a fresh value " P$'$ " on that given dat, and the weighing operation of the apparatus by the use of the load cell 13a, i.e., steps 5 and 6, is carried out on that given day by using the value " P$'$ ".

The interrupt routine ( steps 7 through 14 ) is repeated every day until the switch 41a is intentionally turned off.

Referring to Fig. 6, the electric voltage value supplied from the load cell 13a to the A/D converter circuit 22 is converted into a digital value of, for example, 10 bits, and the digital value is sent to the control unit 2. Accordingly, 1,024 different kinds ( $2^{10}$, i.e., 10 bits ) of weights of loads can be distinctly measured by the load cell 13a of the apparatus of the present embodiment. Thus, if a load is digitally measured at every 0.1 Kg, a load reaching 102.4 Kg can be distinctly measured and displayed on the display unt comprising the panel display 320, the printer 31, and the loudspeaker 34. Note, the no load output of the load cell 13z is an electric voltage corresponding to the weight at 2.4 Kg, and the actual measurable weight is up to 100 Kg.

When displaying the weight value, one or two of the panel display 320, the printer 31, and the loudspeaker 34 may be selectively operated by the appropriate switches.

Further, the RAM 29 may be a non-volatile RAM capable of storing the non load reference value of the load cell 13a, and the rewriting of the stored value may be carried out only when the triggering signal is output from the timer 26.

Moreover, in the afore-described operation, the comparing value " JD " means a range $-\infty \sim$ JD, and if needed, a range between $JD_1 \sim JD_2$ may be used for comparison.

From the foregoing description of the embodiment of Figs. 5 through 8, it will be understood that the health care administration and measuring apparatus of this embodiment has an automatically effects a compensation of the no load reference value ( zero point ) of the load cell 13a used as a scale at an periodic time interval without a manual operation. Thus, an accurate weight operation is always ensured.

The health care administration and measuring apparatus according to a further embodiment of the present invention comprises at least one measuring end for measuring health care data of an examined person and using such data as health care parameters, a control unit for calculating measured values with respect to the health care data on the basis of the signals output from the measuring end, and a data display unit for displaying the measured data with respect to the health care data, in the form on visual data, acoustic data, and printed data, a data file for storing the health care data measured by the measuring end, a normal standard range calculating means for calculating and setting a range of normal standard values from a plurality of past measured data with respect to a corresponding measuring item, and a comparing and determining means for comparing the above-mentioned latest measured health care data with the range of normal standard values calculated and set by the standard range calculating means.

Accordingly, whenever measurement of the health care data of a person to be examined is conducted, the measured data is compared with the range of the normal standard values with respect to respective measuring items, and a determination of adequacy of the physical condition and health of the person at that time is provided for the person in the form of a display of information or a voice output.

The above-mentioned data file, the normal standard range calculating means, and the comparing and determining means may be integrally incorporated in the apparatus in the form of a single determining unit. Alternatively, these data file, normal standard range calculating means, and comparing and determining means may be arranged in a general control installation located remote from the health care administration and measuring apparatus. Accordingly, a plurality of these apparatuses may be interconnected with the general control installation by conventional telecommunication lines or telephone lines, to thereby form a health care administration and measuring system.

In the latter case, a determining unit which comprises a group standard range calculating means for calculating and setting a range of group standard values on the basis of measured data of a plurality of persons to be examined with respect to each of the measuring items, and a range of personal standard values on the basis of a plurality of measured data of each person to be examined with respect to each of the measuring items may be constructed, and arranged in the general control installation.

Referring to Fig. 9, a health care administration and measuring system according to an embodiment of the present invention is formed as a health care network system having a determining unit 200 as a general administration unit to which a plurality of health care administration and measuring apparatuses 100 regarded as terminal units, respectively, are connected. Therefore, each health care administration and measuring apparatus, i.e., each terminal unit, is disposed at a user's home, and the users of the respective terminal units may be considered clients of the network system.

The health care administration and measuring apparatus 100 includes an operating unit 104 provided with an identification ( ID ) input unit 104b comprised of either a button switch unit assigned to the names of preliminarily subscribed persons, a combination of cards and a card reader, or ten keys by which ID code numbers ( personal identification numbers ) are input, a measuring item input unit 104a comprising either button switches, a combination of cards and a card reader, or ten keys for inputting ID numbers, and used for selecting or inputting measuring items for health care administration, such as blood pressure, electrocardiogram, or urinalysis, a plurality of measuring ends 101 to measure health care data of such measuring items, a control unit 102 comprising a CPU, a ROM, and a RAM and electronically processing the measured health care data, an outer connecting circuit 106 comprising a signal transmitting unit 106a for transmitting the clients' codes, ID codes, measuring item codes, and the measured data, and a signal receiving unit 106b for receiving signals sent from a later-described determining unit 200 of the general administration installation, and a display unit 103 comprising a printer, an acoustic synthesizer, and an LCD visual display.

The determining unit 200 receives all data and signals transmitted from the plurality of the above-mentioned health care administration and measuring apparatuses 100, and files the data in association with respective ID codes and respective measuring items, to calculate personal and group standard ranges in association with age and sex, to determine physical conditions and health of the examined persons ( respective clients ) on the basis of their measured data, and to transmit the results of the determinations to corresponding clients. The determining unit 200 is an electronic computer aided unit, and is installed in the general administration installation. When the determining unit 200 is connected with respective health care administration and measuring terminal units by utilizing conventional telephone lines, the signal and data exchanges are conducted via an exchange machine 150.

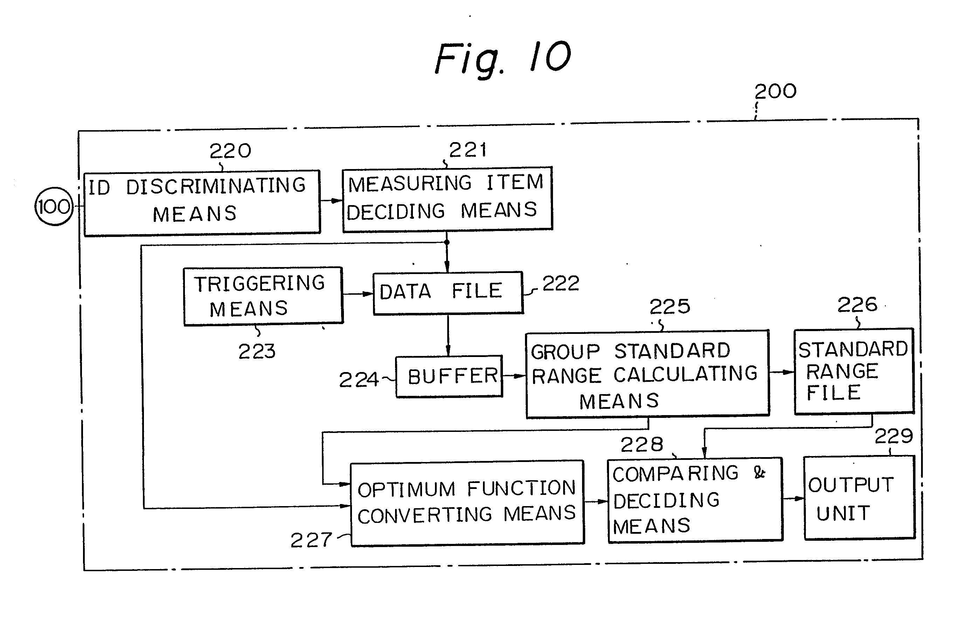

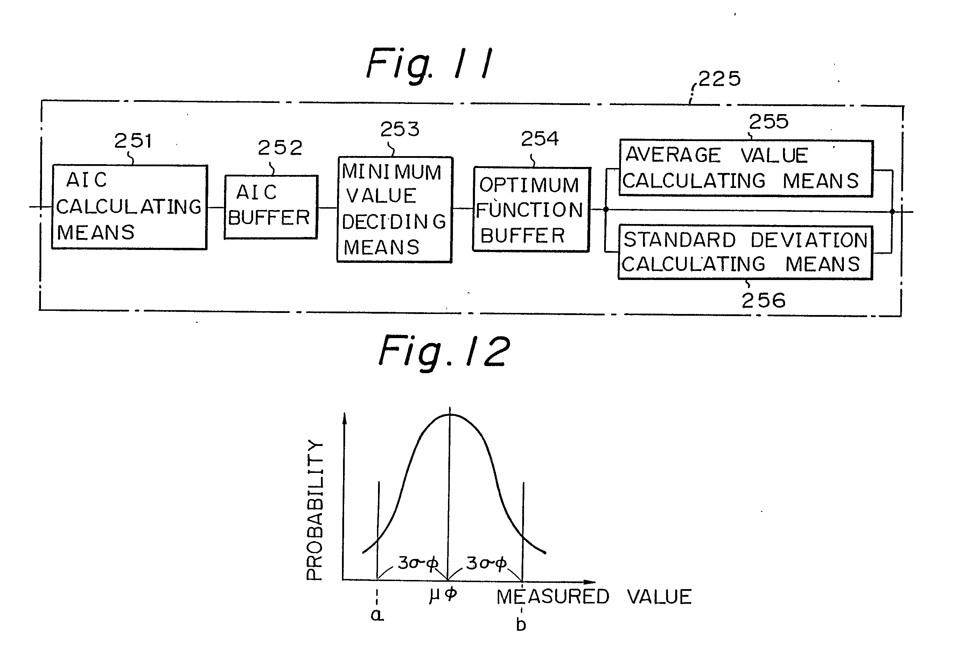

Referring to Figs. 10 and 11, which illustrate the internal arrangement of the determining unit 200, the unit 200 has an ID discriminating means 220 for discriminating whether or not ID codes of respective health care data measured with respect to each measuring item of each client and sent from the client's health care administration and measuring apparatus ( .the terminal unit ) 100 correspond to preliminary registered ID codes ( a contract code plus a personally assigned code ) with respect to that client, a measuringitem deciding means 221 for deciding whether or not the measured and received dada include preliminarily registered codes for respective measuring items, a data file 222 for filing respective measured and received data in association with the time set in that data by a calendar watch function of each terminal unit and the code of each measuring item, a triggering means 223 for generating a triggering signal to start a calculation of the standard ranges when either a predetermined number of the measured health care data is collected or the lapse of a predetermined time is confirmed, a buffer 224 for temporily storing the data read out of the data file 222 to permit the calculation of the read data, a group standard range calculating means 225 for calculating a group standard range for each measuring item, a standard range file 226 for storing the group standard ranges of respective measuring items in association with age and sex, an optimum function converting means 227 for conducting a conversion processing to multiply the measured data sent from each apparatus 100 ( terminal unit ) by an optimum function$\phi$ calculated by the group standard range calculating means 225, a comparing and deciding means 228 for deciding the data processed by the use of

the optimum function $\phi$ by the optimum function converting means 227, and an output unit 229 for controlling the output of the result of the decision by the comparing and deciding means 228, to thereby register the results of the decision in an administration file ( not shown ) of the general administration installation, print the result of the decision, and inform the client of the results of the decision.

The description of the group standard range calculating means 225 will now be provided with reference to Fig. 11.

The group standard range calculating means 225 is arranged for predicting and setting a highly accurate group standard range by obtaining a normal distribution from the measured primitive data. In order to obtain the normal distribution, the group standard range calculating means 225 executes a logarithmic conversion and an exponential conversion by employing the optimum function $\phi$.

In AIC calculating means 251 of the group standard range calculating means 225 is arranged, to obtain the optimum function $\phi$, by executing the calculation of the known Akaike Information Criterion. The AIC calculating means 251 is connected to an AIC buffer 252 on the subsequent stage, which is provided for temporarily storing the results of the optimum function obtaining calculation executed by the AIC calculating means 251. The AIC buffer 252 is connected to a subsequent minimum value deciding means 253 which is arranged to decide which of the results of the calculation stored in the AIC buffer 252 is the minumum and acquire the optimum function $\phi$ at the minimum calculation result. The minimum value deciding means 253 is connected to a subsequent optimum function buffer 254 for temporarily storing the optimum function $\phi$ at the minimum calculation result acquired by the minimum value deciding means 253.

The optimum function buffer 254 is connected to both an average value calculating means 255 for obtaining an average value $\mu \phi$ by using the optimum function $\phi$ at the minimum calculation result, and a standard deviation calculating means 256 for calculating a standard or normal deviation $\sigma \phi$.

The operation of the above-mentioned health care administration and measuring system will be provided below.

An examined person ( a client ) who wishes to be subjected to health care administration presses a button switch of the ID input unit 104b ( Fig. 9 ) of the terminal unit ( the health care administration and measuring apparatus ) 100. The button switch of the ID unit 104b is assigned to, for example, the name of that person. When the control unit 102 receives the signal ( a contract code plus a personal code ) due to the pressing of the button switch of the the ID input unit 104b, the measuring item input unit 104a is brought to a stand-by condition to receive inputs in response to a command signal generated by a control unit 102. Therefore,the person inputs an desired measuring item or items from the measuring item input unit 104a. Namely, when desiring to measure, for example, the blood pressure, the person presses the button switch of the measuring item input unit 104a assigned to blood pressure. When the control unit 102 receives the measuring item input signal sent from the unit 104a, the unit 102 connects an electric power ( not shown ) to a measuring end or measuring ends 101 to bring the measuring ends into a stand-by condition to commence a measuring operation.

The measuring ends 101 for measuring a blood pressure carry out the measurement of the basis of the known priciple of the afore-mentioned photoelectric plethysmographic mrthod. When an electrocardiogram is measured, measuring ends 101 in the form of cardiographic electrodes are used, and attached to the breast region of a person to be measured to measure the electrocardiogram.

As described, when the measured person selects the blood pressure as the desired measuring item, the measured data of the measuring end 101 is sent from the signal transmitting unit 106 via the control unit 102 to the determining unit 200 via the exchange machine 150, in the form of the client's code signal including a contract code, a personal code, a measuring item code, and measured data.

The determining unit 200 in turn receives the transmitted client's code signal through a receiving unit thereof ( not shown ), and discriminates whether or not the contract code and the personal code correspond to the preliminary registered contract and personal codes, by the ID discriminating means 220. Subsequently, the measuring item code of the received transmitted client's code signal is checked by the measuring item deciding means 221.

When a complete correspondence between the client's code sent from the terminal unit 100 and the preliminarily registered codes of the determining unit 200 is not obtained during the operation of the ID discriminating means 220 and the measuring item deciding means 221, all data sent from the terminal unit 100 is returned from the output unit 229 of the determining unit 200 to the corresponding terminal unit 100 while the client is informed that an abnormal state exists in the operation of the terminal unit 100, through the telephone lines.

When a complete correspondence between the client's code sent from the terminal unit 100 and the preliminarily registered codes of the determining unit 200 is obtained during the operation of the ID discriminating means 220 and the measuring item deciding means 221, the client's code including the measured health care data is filed in a corresponding measuring item file of addressed personal files ( the client's age and sex are preliminarily registered in the addressed files ) of the data file 222 by writing the time and calendar day added by the terminal units 100. The measured health care data in the client's code are also sent to the comparing and deciding means 228 via the optimum function converting means 227, for a comparing and deciding operation.

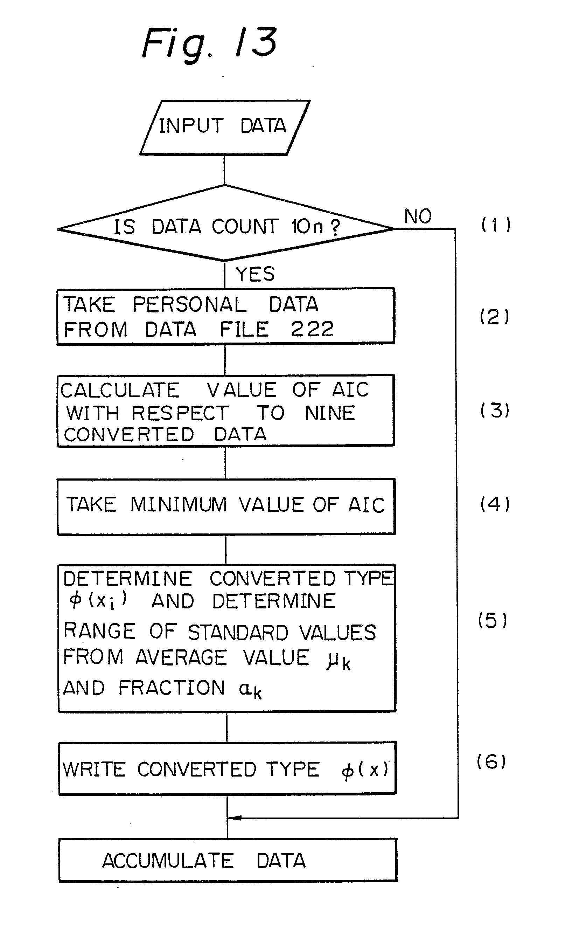

Referring to Fig. 13 illustrating the flow chart of the operation of the determining unit 200, the triggering means 223 accesses the data file 222 when the measured health care data received from the terminal units 100 reaches a predetermined number, for example, 2, 000 data with respect to age and sex, or when a predetermined time is reached ( for example, one o'clock a.m. every Monday ) ( step 1 ), and takes the latest personal data of each person with respect to each measuring item and in association with the person's age and sex, out of the data file 222 one after another, except for data deviating from the corresponding standard ranges. Then, the taken data is temporarily stored in the buffer 224 ( step 2 ), and the stored data is then processed by the group standard range calculating means 225 to obtain respective ranges of group standard values. The AIC calculating means 251 calculates the value of AIC ( Akaike Information Criterion ) in accordance with the equation set forth below.

$$\text{AIC } ( \phi , \text{ k,m } ) = \text{ n } ( \log 2 \pi \sigma^2 + 1 )$$

$$-2 \sum_{i=1}^{n} \log | \phi' ( X_i ) |$$
$$-2 \log (n-k-m) !$$
$$+2 ( k+m+2+s )$$

$\phi$ : conversion types ( $X^{-3}$ , $X^{-2}$, $X^{-1}$, $X^{1/3}$ , $X^{1/2}$ , $X < \log X$ , $X^2$ , $X^3$ )
k : presumed number of defective data in the lower region of the data
m : presumed number of defective data in the upper region of the data
n : number of all data
$\sigma$ : standard deviation

$$\sigma = \sqrt{\frac{\sum_{i=1}^{n} \{ \phi ( X_i ) - \mu \}^2}{n - 1}}$$

$\mu$ : average value

$$\mu = 1 / n \cdot \sum_{i=1}^{n} \phi ( X_i )$$

s : constant ( an exponential conversion: s = 1,non exponential conversion : s = 0 )

As a result of the above-mentioned calculation, the value of AIC is registered in the AIC buffer 252. Note: nine results depending on nine conversion types are obtained. Subsequently, the minimum value deciding means 253 decides the minimum valie of the nine AIC values ( step 4 ), and registers the resultant optimum function $\phi$ corresponding to the minimum AIC value in the optimum function buffer 254. By multiplying the primitive data by the obtained optimum function$\phi$ , it is possible to bring the primitive data close to a standard or normal deviation.

Subsequently, the average value calculating means 255 calculates an average value$\mu$ $\phi$ shown in Fig. 12 by using the calculated optimum function$\phi$ above. Also, the normal deviation calculating means 256 calculates a normal deviation $\sigma\phi$ by the use of the calculated optimum function$\phi$ . At this stage, since during the above-mentioned calculation of the AIC values, the AIC calculating means 251 has already calculated the average value$\mu\phi$ and the normal deviation $\sigma$ $\phi$, these calculated values may be alternatively registered in the average value calculating means 255 and the standard deviation calculating means 256, respectively.

From the foregoing steps, the range of the group standard values are decided. Figure 12 illustrates the range of the group standard values spanning the values " a " and " b " , i.e., the range of $\pm 3 \sigma \phi$ with respect to the central average value $\mu\phi$ (step 5).

It should be understood that, when the range is $\mu\phi \pm 3 \sigma\phi$, 99.74 % of the primitive data will be within this range from the statistical view point. Nevertheless, a constant other than " 3 " may be used depending on the measuring item, the method of measuring the health care data, and the accuracy of the measurement.

The range of the standard values between a and b of Fig. 12 is written in the standard range file 226 ( Fig. 10 ) at step 6.

In the case of blood pressure, the maximum and minimum values of the blood pressure are always measured, and therefore, two ranges of standard values must appear.

It should be noted that the decision of the range of the standard values is made for each measuring item while taking the age and sex of the person into consideration. Namely, a plurality of ranges of the standard values are decided.

As described before, the measured health care data sent from the terminal unit 100 is input to the comparing and deciding means 228 of the determining unit 200 via the optimum function converting means 227. On the other hand, the ranges of the standard values of the measuring item with respect to respective persons' age and sex are simultaneously input to the comparing and deciding means 228 in relation to the above-mentioned measured health care data from the terminal unit 100, and therefore, the comparing and deciding means 228 executes the deciding operation to determine whether or not the measured health care data sent from the terminal unit 100 converted into the optimum function is within the range of the standard values.

When the data is decided to be outside the range of the standard values, a signal is sent to the corresponding terminal unit 100 for promoting the display unit 103 of that terminal unit 100 to indicate that the measurement should be tried again, taking into the account that either the terminal unit 100 is in an abnormal condition or the unit 100 has made an erroneous previous measurement.

When the measurement of the terminal unit 100 is conducted again, and when the measured data is again sent to the determining unit 200, the comparing and deciding means 228 of the determining unit 200 conducts a deciding operation similar to that conducted previously. Then, if the data is still outside the range of the standard values, the determining unit 200 sends a signal to the corresponding terminal 100 to indicate on the display unit 103 the comment --- You ( the measured person ) should consult a medical doctor. ---.

Although the above-mentioned indication is conducted when two deciding operations show that the data is outside the range of the standard values, the number of deciding operations may be increased depending on the reliability of the respective terminal units 100.

The above-mentioned method of advising the client person to consult a medical doctor at the early stage of an illness by the health care administration system of the present embodiment makes it possible to attain a satisfactory administration of the health care of each client person.

When the comparing and deciding operations of the comparing and deciding means 228 of the determining unit 200 indicate that the latest measured data of the measured person is within the range of the standard values, the display of --- Your latest data is within the range of the standard values.--- is made on the display unit 103, to give the client a feeling of relief.

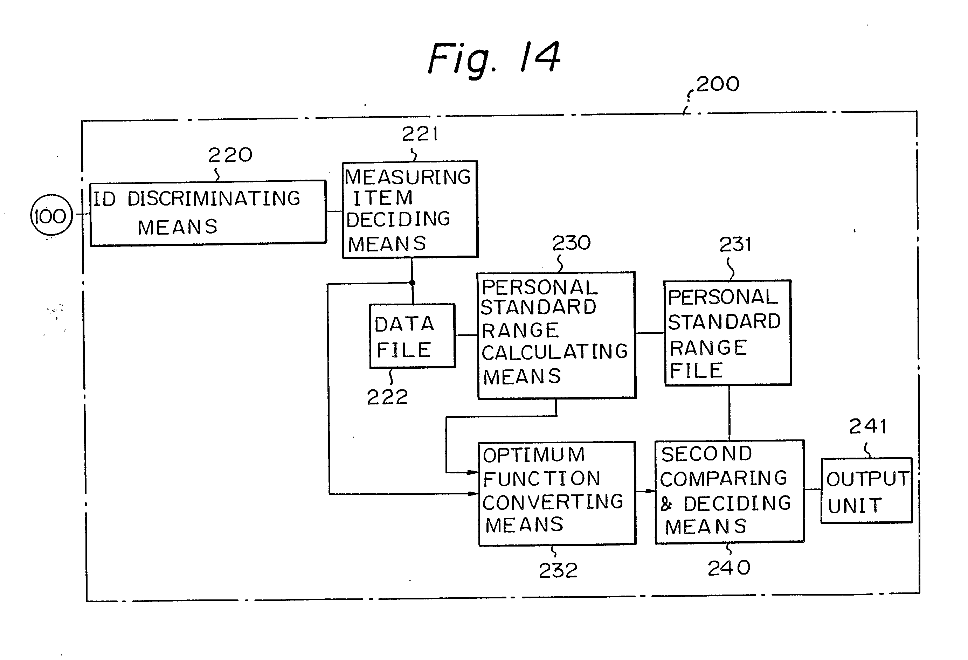

Figure 14 illustrates another embodiment of the determining unit 200 of the general administration installation.

The difference between the present embodiment and that of Fig. 10 is that, in the triggering means 223, the buffer 224 of the embodiment of Fig. 10 is omitted, and that every time the client's data including the measured health care data is input to the determining unit 200 of the general administration installation, a range of standard values is set in the determining unit 200. Nevertheless, the range of standard values may be alternatively set in the determining unit 200 when a predetermined number of measured data ( for example, 20 data ) of the client are input to the determining unit 200.

A personal standard range calculating means designated at 230 has a means having a fuction similar to that of the group standard range calculating means 225 of Fig. 10, and a personal standard range file 231 is arranged for filing a range of personal standard values of each person, which are calculated by the personal standard range calculating means 230. Designated at 232 is an optimum function converting means, which is the same as the optimum function converting means 227 of Fig. 10, and at 240 is a second comparing and deciding means for comparing the measured health care data sent from each of the terminal units 100 and converted into an optimum function within the range of the person standard values ( the range between the values " c " and " d " ) filed in the personal standard range file 231, and decides whether or not the measured health care data is within the range of the personal standard values. The function of the second comparing and deciding means 240 is similar to the comparing and deciding means 228 of Fig. 10. A output unit 241 is arranged for transmitting the result of the determining unit 200 to the display unit 103 of the corresponding terminal unit 100.

When the range of the group standard values is set and used, the data should be evenly collected from many clients constituting a group of persons to be examined, without a prejudiced collection of the data. Therefore, the embodiment of Fig. 10 is preferably employed.

The setting of the range of the personal standard values is preferably made in such a manner that the measured health care data is input to the determining unit 200, from the view point of a high accuracy of the calculation of the range of the personal standard values. Thus, the embodiment of Fig. 14 is employed. When only the range of the personal standard valus is employed for comparing and deciding the measured health care data of a person to be examined, if the arrangement similar to that shown in Fig. 14 is provided in each terminal unit 100, it is not necessary to sent the data to the determining unit 200 to achieve the necessary determining operation to decide the adequacy of each person's health and physical condition.

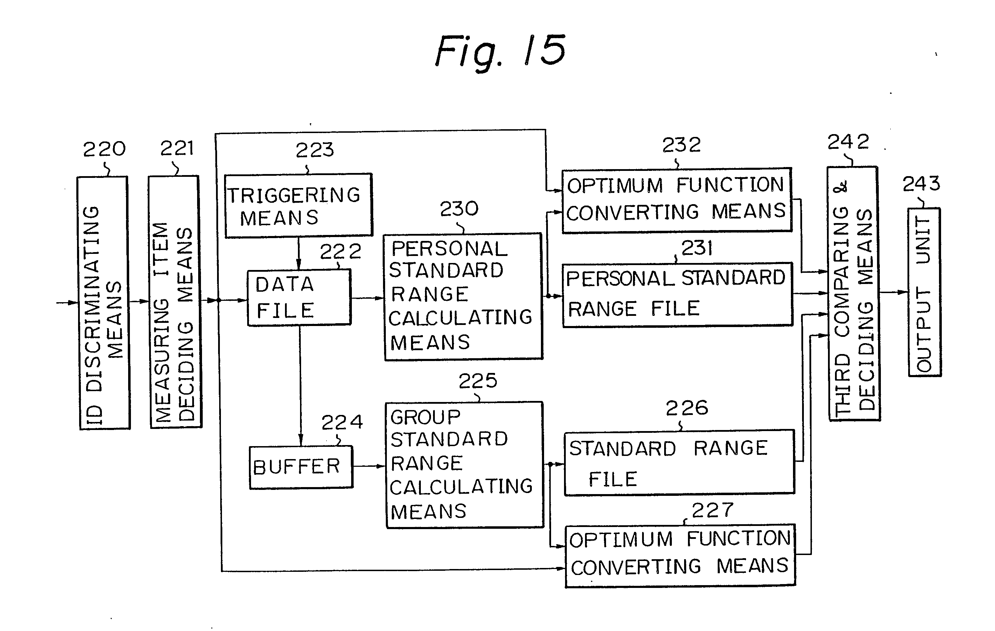

Figure 15 illustrates the embodiment wherein both ranges of the group and personal standard values are employed for determining the physical condition and health of each person.

Referring to Fig. 15 illustrating an arrangement of a determining unit of the general administration installation, it should be understood that the same reference numerals as those of the former embodiments of Figs. 10 and 14 designate the same or like units and functional means.

Reference numeral 242 is a third comparing and deciding means for conducting the comparing and deciding operation on the basis of the measured health care data converted with the optimum functions of both ranges of the group and personal standard values and the ranges of the group and personal standard values, and 243 is an output unit for transmitting the results of the comparing and deciding made by the third comparing and deciding means 242.

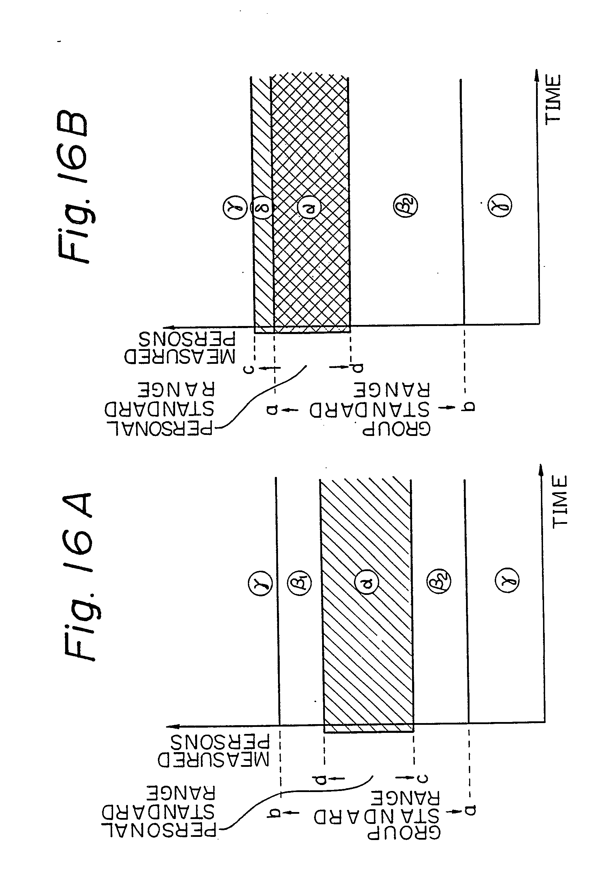

The operation of the embodiment of Fig. 15 will be provided with reference to Figs. 16A and 16B in addition to Fig. 15.

The physical condition and health of each person are different, and therefore it is well known that the health care data of some persons are stable at relatively high values and those of other persons are stable at relatively low values.

When consideration is given to both ranges of the group and personal standard values, the width of the range of the group standard values on the basis of non-specified persons' data is statistically larger than that of the personal standard values, as illustrated in Fig. 16A.

Nevertheless, although statiscally small, the maximum value ( c ) of the range of the personal standard values occasionally rises above the maximum value ( d ) of the range of the group standard values, as shown in Fig. 16B, when the data is stable at relatively high values. Also, there is a case where the range of the personal standard values falls below the maximum value of the range of the group standard values when the data is stable at relatively low values.

The operation of the embodiment of Fig. 15 will be described.

First, the ranges designated by $\alpha$ through $\delta$ in the graphs of Figs. 16A and 16B illustrating the relationship between the range of the group normal standard values ( the ordinate ) and the time ( the abscissa ) will be explained. " $\alpha$ " indicates a range within both ranges of the group and personal standard values. " $\beta$ " indicates ranges within the range of the group standard values and outside the range of the personal standard values " $\tau$ " indicates a range outside both the ranges of the group and personal standard values. " $\delta$ " indicates a range outside the range of the group standard values and within the range of the personal standard values.

When the measured health care data of a measured person sent from a corresponding terminal unit 100 ( Fig. 9 ) is in the range $\alpha$, the third comparing and deciding means 242 of Fig. 15 decides that the sent health care data is within both the ranges of the group and personal standard values, and therefore, the output unit 243 transmits a signal indicating -- the data is within the ranges. -- to the terminal unit 100 where the display unit 103 ( Fig. 9 ) displays same.

When the measured health care data of the measured person sent from the terminal 100 is in the range $\beta$, the range $\beta_1$ is above the range of the personal standard values, but within the range of the group standard values. The range $\beta_2$ is below the range of the personal standard values and within the range of the group standard values. Therefore, the display of a statement --- You should measure again tomorrow. --- or --- You should be careful.--- is made on the display unit 103. If the result of the decision on the next day also is in the range $\beta$, a statement --- You should consult a medical doctor. ---is displayed on the display unit 103 of the terminal unit 100.

When a change from $\beta_1$ to $\beta_2$ or vice versa occurs next day, the afore-mentioned statement --- You should measure again tomorrow. ---is again displayed on the display unit 103 of the terminal unit 100.

When the measured health care data of the client sent from the terminal unit 100 is in the range $\tau$, the output unit 243 sends a signal to the terminal unit 100 to show a statement --- You should consult a medical doctor.--- displayed on the display unit 103.

When the measured health care data of the client sent from the terminal unit 100 is in the range $\delta$, the display of the statement ---You should measure again tommorow. --- or --- You should be careful. --- is carried out by the display unit 103. When this decision is repeated thrice, the statement ---You should consult a medical doctor.--- is displayed on the display unit 103. Thus, the health care data is subjected to a determination of the adequacy of the physical condition and health of the client on the basis of both the ranges of the group and personal standard values,and accordingly, the determination is accurate.

According to the above-mentioned health care administration system, the actually measured updated health care data is used for setting the range of ther personal and/or group standard values. The determination of the adequacy of the health and physical condition of respective persons is always highly accurate. Since when the measured data of a person indicates that the person may be ill, the statement advising --- You should consult a medical doctor--- is displayed on the display unit, and thus an early detection of an illness can be achieved.

Further, since a person carries out the measuring of his or her health care data at home, it is possible for the person to administer an own health care and to easily supply a medical doctor with valuable data for medical examinations.

In addition, since the latest measured health care data are compared with the past collected personal and group health care data, to decide each person's physical condition and health, a high quality administration of the health care can be ensured.

It should be understood that many variations and alterations will occur to persons skilled in the art within the scope and spirit of the present invention set forth in the appended claims.

## Claims

1. A health care administration apparatus capable of measuring at least one health care data used as a parameter for examining a physical condition and health of a person to be examined comprising :
an operating means for selecting measuring items to be employed as said health care data for said person and setting same into said apparatus;
a measuring means interconnected with said operating means, and including a measuring end for measuring said health care data and generating measured data output signals in response to a setting signal issued from said operating means;
a control means for calculating measured values with respect to said health care data on the basis of said signals output from said measuring means;
a data display means for displaying said measured data with respect to said health care data to at least said person under examination; and
a support means for supporting said person to be examined, in a posture thereof adapted for being measured, said support means including a constructional framework member for accommodating and incorporating therein said operating, measuring, control, and data display means.

2. A health care administration apparatus as claimed in claim 1, wherein said measuring means includes a plurality of measuring ends adapted for carrying out measurements of at least an e lectrocardiogram, weight, and a blood pressure of said person to be examined.

3. A health care administration apparatus as claimed in claim 1, wherein said control means comprises an input terminal connected to said measuring end of said measuring means, and output terminals connected to said operating means and said data display means, respectively.

4. A health care administration apparatus as claimed in claim 1, wherein said data display means comprises a visual panel display, a loudspeaker, and a printing unit.

5. A health care administration apparatus as claimed in claim 4, wherein said visual panel display of said data display means has a panel display on which results of measurement of said measuring end of said measuring means are displayed.

6. A health care administration apparatus as claimed in claim 1, wherein said operating means comprises a group of switching members to be operated for selecting one operation mode from a plurality of operation modes.

7. A health care administration apparatus as claimed in claim 1, wherein said constructional framework member of said support means includes an armchair-like framework member comprising a seat, a pair or arms, a backrest, and a base plate, said backrest accommodating said control means therein.

8. A health care administration apparatus as claimed in claim 7, wherein said arms and said base plate of said support means accommodate therein said measuring ends of said measuring means for conducting elecrocardiogram and blood pressure measurements.

9. A health care administration apparatus as claimed in claim 7, wherein said base plate of said support means accommodates therein said measuring end of said measuring means for conducting weight measurements.

10. A health care administration apparatus as claimed in claim 9, wherein said measuring end of said measuring means for conducting measurements of weight comprises a load cell including a strain gauge element therein.

11. A health care administration apparatus as claimed in claim 10, wherein said apparatus further comprises :

a means for generating, at each lapse of a predetermined time, a trigger signal to trigger a commencement of an operation to compensate for zero point of said load cell;

a zero point compensation deciding means for deciding whether or not a differential between a value of a present output output from said load cell and a reference measuring value under no load and presently stored in a reference measuring value memory means as a value of a zero point of said load cell is kept in a predetermined range each time said trigger signal is generated by said means for generating a trigger signal;

a rewriting means for rewriting said memory means with said value of the present output from said load cell when said zero point compensation deciding means decides that said differential is within said predetermined range; and

a weight calculating means for calculating an actual value of a weight of a load to which said load cell is subjected by calculating a difference between a measuring signal generated from said load cell during the subjecting of said load and said reference measuring value, while said trigger signal is not generated by said means for generating a trigger signal.

12. A health care administration apparatus as claimed in claim 11, wherein said control means comprises a central process unit ( CPU ) functioning as said zero point compensation deciding means, said rewriting means, and said weight calculating means, and a random memory unit ( RAM ) functioning as said reference measuring value memory means.

13. A health care administration apparatus as claimed in claim 1, wherein said apparatus further comprises :

a transmitting circuit means for transmitting said measured values with respect to said health care data from said control means to outside of said health care administration apparatus; and

a receiving circuit means for receiving a result of measurements of health care data from outside health care administration apparatus.

14. A health care administration apparatus as claimed in claim 1, wherein said apparatus further comprises a determining means for determining an adequacy of the physical condition and health of said person to be examined on the basis of said measured values with respect to said health care data calculated by said control means, said determining means comprising:

a data file for storing said measured values with respect to said health care data;

a standard range calculating means for calculating and deciding a range of standard values from a plurality of measured values of said person to be examined with respect to said selected measuring items; and

a comparing and determining means for comparing the latest of said measured values with said range of standard values calculated by said standard range calculating means.

15. A health care administration system for conducting health care of a person to be examined by measuring more than one health care datum used as a parameter of a physical condition and health of the person, comprising:

at least one terminal health care administration and measuring unit which comprises an operating means for selecting measuring items to be employed as said health care data for said person and setting same into said apparatus, a measuring means interconnected with said operating means, and including a measuring end for measuring said health care data and generating measured data output signals in response to a setting signal issued from said operating means, a control means for calculating measured values with respect to said health care data on the basis of said signals output from said measuring means, and a data displaying means for display said measured data with respect to said health care data to at least said person to be examined;

a determining means for determining an adequacy of the physical condition and health of said person to be examined on the basis of said measured values with respect to said health care data calculated by said control means of said terminal health care administration and measuring unit, said determining means comprising:

a data file interconnected with said terminal health care administration and measuring unit and storing said measured values with respect to each of said selected items;

a standard range calculating means for calculating and setting a range of standard values from a plurality of measured past values of said person to be examined with respect to each of said selected measuring items; and

a comparing and determining means for comparing the latest of said measured values with said range of standard values calculated and set by said standard range calculating means.

16. A health care administration system according to claim 15, wherein said determining means is located at an outer health care center interconnected with a plurality of terminal health care and measuring units through intercommunication lines.

17. A health care administration system according to claim 15, wherein said standard range calculating means of said determining means comprises a computing means for carrying out a computation of Akaike Information Criteria.

18. A health care administration system according to claim 15, wherein said standard range calculating means of said determining means comprises:

a group standard range calculating means for calculating a range of group standard values on an equal measuring item from said measured values with respect to said health care data on the equal measuring item for a plurality of persons;

a personal standard range calculating means for calculating a range of personal standard values on each measuring item for each of said plurality of persons from a plurality of measured past values on said each measuring item; and

a comparing and determining means for comparing the latest measured value on said each item for said person with said range of group standard values and said range of personal standard values on said each measuring item for said person.

NHH-7105-EP

# Fig. 1

# Fig. 2

# Fig. 3

EP 0 329 306 A1

## Fig. 4 (b)

### AT POSITION USED AS SCALE

## Fig. 4 (a)

### AT UNUSED POSITION

15
30c
30b
30a
11
30

15
30a
30b
11
11
30d

## Fig. 4 (c)

### AT SETTING POSITION

## Fig. 4 (d)

### AT MEASURING POSITION

15
30a
30b
30c
11
30d

15
30b
30c
30
11
30d

# Fig. 5

# Fig. 6

## Fig. 7

```
ELECTRIC POWER      ⌐1
IS MADE "ON"
        │
READ    "ST"        ⌐2
        │
WRITE   "ST"        ⌐3
IN RAM
        │
READ    "DA"        ⌐4
        │
READ  "ST(P)"       ⌐5
OUT OF RAM
        │
W = DA − ST         ⌐6
        │
PANEL
DISPLAY             ⌐320
        │
PRINTER             ⌐31
        │
LOUD-
SPEAKER            ⌐34
```

## Fig. 8

```
INTERRUPTING        ⌐7
STEPS
        │
READ    "P"         ⌐8
        │
READING OUT         ⌐9
"JD"
        │
READ "ST(P)"        ⌐10
OUT OF RAM
        │
ΔD = P − ST(P)      ⌐11
        │
        12
ΔD ≦ JD ?  ───NO──┐
        │         │
       YES        │
        │         │
REWRITE "ST(P)"   │ ⌐13
OF RAM            │
WITH "P"          │
        │◄────────┘
        │
END                 ⌐14
```

Fig. 9

EP 0 329 306 A1

## Fig. 10

EP 0 329 306 A1

# Fig. 11

251 — AIC CALCULATING MEANS
252 — AIC BUFFER
253 — MINIMUM VALUE DECIDING MEANS
254 — OPTIMUM FUNCTION BUFFER
255 — AVERAGE VALUE CALCULATING MEANS
256 — STANDARD DEVIATION CALCULATING MEANS
225

# Fig. 12

PROBABILITY

$3\sigma\phi$   $3\sigma\phi$

$\mu\phi$   MEASURED VALUE

a   b

EP 0 329 306 A1

# Fig. 13

INPUT DATA

IS DATA COUNT 10n ?  — NO — (1)

YES

TAKE PERSONAL DATA
FROM DATA FILE 222 — (2)

CALCULATE VALUE OF AIC
WITH RESPECT TO NINE
CONVERTED DATA — (3)

TAKE MINIMUM VALUE OF AIC — (4)

DETERMINE CONVERTED TYPE
$\phi(x_i)$ AND DETERMINE
RANGE OF STANDARD VALUES
FROM AVERAGE VALUE $\mu_k$
AND FRACTION $\alpha_k$ — (5)

WRITE CONVERTED TYPE $\phi(x)$ — (6)

ACCUMULATE DATA

EP 0 329 306 A1

## Fig. 14

- 200
- 221 MEASURING ITEM DECIDING MEANS
- 220 ID DISCRIMINATING MEANS
- 100
- 230 PERSONAL STANDARD RANGE CALCULATING MEANS
- 231 PERSONAL STANDARD RANGE FILE
- 222 DATA FILE
- 232 OPTIMUM FUNCTION CONVERTING MEANS
- 240 SECOND COMPARING & DECIDING MEANS
- 241 OUTPUT UNIT

# Fig. 15

220 ID DISCRIMINATING MEANS

221 MEASURING ITEM DECIDING MEANS

223 TRIGGERING MEANS

222 DATA FILE

224 BUFFER

230 PERSONAL STANDARD RANGE CALCULATING MEANS

225 GROUP STANDARD RANGE CALCULATING MEANS

232 OPTIMUM FUNCTION CONVERTING MEANS

231 PERSONAL STANDARD RANGE FILE

226 STANDARD RANGE FILE

227 OPTIMUM FUNCTION CONVERTING MEANS

242 THIRD COMPARING & DECIDING MEANS

243 OUTPUT UNIT

EP 0 329 306 A1

Fig. 16A

Fig. 16B

European Patent Office

**EUROPEAN SEARCH REPORT**

Application Number

EP 89 30 1029

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | US-A-4 105 021 (W.J. WILLIAMS et al.)<br>* Abstract; column 5, lines 41-58; figures 1,2,5 * | 1,3 | A 61 B 5/02 |
| Y | | 2,6,7,9 | |
| A | | 4,5 | |
| X | US-A-4 312 359 (T.A. OLSON)<br>* Abstract; column 4, line 65 - column 5, line 14; figures 1-3 * | 1,13 | |
| Y | | 7,9 | |
| A | | 14 | |
| Y | US-A-4 216 462 (W.J. McGRATH et al.)<br>* Abstract; column 2, line 38 - column 3, line 27; figures 1-8 * | 2,6 | |
| A | | 8,15,16 | |
| A | US-A-4 033 336 (F.H. MURAWSKI et al.)<br>* Abstract; figures 1,2,10-14 * | 1-3,9,<br>10,15 | |
| A | US-A-4 313 510 (H.W. TOMLINSON, Jr.)<br>* Abstract; figure 8 * | 9-12 | TECHNICAL FIELDS SEARCHED (Int. Cl.4)<br><br>A 61 B |

The present search report has been drawn up for all claims

| Place of search | Date of completion of the search | Examiner |
|---|---|---|
| THE HAGUE | 11-05-1989 | HUNT,B.W. |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons
 
& : member of the same patent family, corresponding
    document

EPO FORM 1503 03.82 (P0401)